# EUROPÄISCHE PATENTSCHRIFT

(11) **EP 1 052 949 B1**
(45) Veröffentlichungstag und Bekanntmachung des Hinweises auf die Patenterteilung: **28.05.2003**
(21) Anmeldenummer: 98965817.4
(22) Anmeldetag: 12.12.1998
(51) Int. Cl.: A61F 2/00

(54) **HÜFTGELENKSPFANNE**
HIP JOINT SOCKET
CAVITE COTYLOIDE

(30) Priorität: 13.12.1997 DE 19755536
(43) Veröffentlichungstag der Anmeldung: 22.11.2000
(73) Patentinhaber: CeramTec AG Innovative Ceramic Engineering, 73207 Plochingen (DE)
(72) Erfinder: VON CHAMIER, Wilfried, D-70193 Stuttgart (DE); KÄLBERER, Hartmut, D-73269 Hochdorf (DE); PFAFF, Hans-Georg, D-73760 Ostfildern (DE)
(74) Vertreter: Uppena, Franz, Dr.
(86) Internationale Anmeldenummer: EP9808110
(87) Internationale Veröffentlichungsnummer: WO99030634

(56) Entgegenhaltungen:
- EP-A- 0 013 863
- EP-A- 0 053 794
- WO-A-97/31592
- DD-A- 298 737
- DE-A- 3 711 426
- DE-A- 3 722 410

## Beschreibung

Die Erfindung betrifft eine Hüftgelenkspfanne einer Hüftgelenk-Endoprothese entsprechend dem Oberbegriff des ersten Anspruchs. Eine Hüftgelenkspfanne gemäß dem oberbegriff von Anspruch 1 ist beispielsweise aus der DE-A-37 22 410 bekannt.

Hüftgelenk-Endoprothesen sind in der Regel modular aufgebaut. Sie bestehen beispielsweise aus einem Schaft, der in den Oberschenkelknochen eingesetzt wird. Auf den Schaft wird ein Kugelkopf aufgesteckt, der in einer aus zwei Teilen aufgebauten Pfanne gelagert wird. Die Pfanne besteht aus einem Pfannengehäuse, dem sogenannten Metal-back, und einem Pfanneneinsatz, dem sogenannten Insert. Es gibt auch noch Systeme, die noch mehr Kombinations- und Variationsmöglichkeiten zulassen, beispielsweise zum Verlängern des Schafts. Bei den modular aufgebauten Endoprothesen werden Implantatkomponenten unterschiedlicher Materialien und Größen miteinander verbunden. Beispielsweise werden Kugelköpfe aus Kobalt-Chrom auf einen Schaft aus Titanlegierung gesteckt oder ein Pfanneneinsatz aus Polyäthylen oder einem keramischen Werkstoff in ein Pfannengehäuse eingesetzt, wie es beispielsweise aus der DE 196 112 48 A1 bekannt ist. Die Kombination der einzelnen Teile wird in der Regel durch die Gelenkabmessungen vorgegeben.

Wie aus der Veröffentlichung "Frettingkorrosion, ein Problem bei Hüftendoprothesen" von G. Willmann, Praktische Orthopädie, Rheumatologie-Endoprothetik, Band 27, 1997, bekannt ist, ist bei dem modularen Aufbau der Endoprothesen nachteilig, daß nach dem Zusammensetzen durch die Belastungen im Körper Lockerungen auftreten können, die zur Relativbewegung zwischen den einzelnen Protheseteilen führen und damit Verschleiß hervorrufen. Darüber hinaus besteht auch aufgrund der Vielzahl der möglichen Komponenten und deren Zusammensetzungsmöglichkeiten die Gefahr einer Verwechslung hinsichtlich der Größen oder einer nicht bestimmungsgerechten Kombination von Prothesenkomponenten. Dieses kann zu Fehlfunktionen der Prothese und damit letztendlich zum Versagen führen.

Aufgabe der vorliegenden Erfindung ist es, eine Hüftgelenkspfanne einer Hüftgelenk-Endoprothese vorzustellen, bei der sowohl eine falsche Zusammensetzung von Pfanneneinsatz und Pfannengehäuse ausgeschlossen als auch eine belastungsbedingte Lockerung der beiden Komponenten verhindert wird.

Die Lösung der Aufgabe erfolgt mit Hilfe der kennzeichnenden Merkmale des ersten Anspruchs. Vorteilhafte Ausgestaltungen der Erfindung werden in den Unteransprüchen beansprucht.

Durch die Erfindung entsteht eine einstückige Hüftgelenkspfanne als implantat. Durch die Beschichtung des keramischen Werkstoffs der Pfanne mit einem biokompatiblen Metall oder einer biokompatiblen Metallegierung in dem Bereich, in dem die Pfanne in den Hüftknochen eingesetzt wird, entsteht eine unlösbare Verbindung zwischen dem Teil der Prothese, das nach dem Stand der Technik zur Lagerung des Kugelkopfs als Einsatz fungiert und dem sogenannten Pfannengehäuse, was die Verbindung zwischen dem Knochen und der Lagerschale für den Kugelkopf herstellt. Dem Chirurgen wird damit nur ein Teil für die Implantation einer Pfanne zur Verfügung gestellt, wobei die Beschichtung optimal auf Größe und Zusammensetzung der keramischen Lagerschale abgestimmt ist. Die Gefahr einer falschen Zusammensetzung von Pfanneneinsatz und Pfannengehäuse ist ausgeschlossen. Weiterhin ausgeschlossen ist die Gefahr, daß sich aufgrund der Belastungen in dem Prothesengelenk die Verbindung zwischen Einsatz und Gehäuse lockert und durch eine Relativbewegung der beiden Komponenten gegeneinander die Prothese verschleißt.

In vorteilhafter Ausgestaltung der Erfindung ist die zu beschichtende Oberfläche der Lagerschale der Hüftgelenkspfanne aufgerauht. Dadurch wird eine innige und feste mechanische Verbindung der Beschichtung mit dem Keramikkörper sichergestellt, da die Beschichtung weder chemisch noch metallurgisch mit der Keramik reagiert. Das Aufrauhen der Oberfläche des Keramikkörpers kann beispielsweise durch Strahlen mit Hartstoffpartikeln, durch Grobschleifen oder Ätzen erfolgen.

Für das Auftragen der Beschichtung stehen insbesondere zwei bekannte und bewährte Verfahren zur Verfügung. Nach dem ersten Verfahren ist die Beschichtung aufgedampft. Das Aufdampfen kann beispielsweise durch Sputtern erfolgen. Dabei wird das aufzutragende Metall im Hochvakuum zerstäubt und scheidet sich aus der Dampfphase auf die Oberfläche-des Substrats ab. Durch mehrfaches Aufdampfen kann eine Beschichtung von der erforderlichen Schichtdicke hergestellt werden.

Die Beschichtung kann auch aufgespritzt sein. Zum Aufspritzen von Metallen oder Metallegierungen, insbesondere von den hochschmelzenden Metallen oder Metallegierungen des Titans, eignet sich das Plasmaspritzen.

Das Plasmaspritzen bietet ebenfalls die Möglichkeit, eine Beschichtung in der erforderlichen Dicke herzustellen. Bei einer Beschichtung in genügender Dicke ist es möglich, die Oberfläche der Beschichtung aufzurauhen. Die Rauhigkeit kann durch das Auftragverfahren oder durch entsprechende Nachbearbeitung so herbeigeführt werden, daß sich dem Knochengewebe die Möglichkeit bietet, mit der Oberfläche zu verwachsen und somit eine sichere Verankerung des Implantats im Knochen zu bewirken. Die Beschaffenheit der Oberfläche kann insbesondere beim Plasmaspritzen bereits durch die Aufspritztechnik erreicht werden, so daß keine Nachbearbeitung erforderlich ist.

Eine besonders gute Verankerung des Implantats im Knochengewebe wird dann erreicht, wenn die Beschichtung porös ist. In diesem Fall kann das Knochengewebe in die Poren hineinwachsen und somit dem Implantat einen besonders guten Halt verschaffen.

Die Dicke der Beschichtung muß so gewählt sein, daß sie einerseits dem Knochengewebe eine genügend große Angriffsfläche zum Verwachsen bietet, andererseits aber nicht in sich bruchgefährdet ist, was beispielsweise bei einer zu dicken porösen Beschichtung der Fall sein kann. Als vorteilhaft hat sich deshalb eine Schichtdicke herausgestellt, die dünner ist als 1 mm. Eine Schichtdicke von 50 Mikrometern bis 150 Mikrometern bietet einerseits die genügende Dicke, um eine für das Einwachsen des Knochengewebes günstige Struktur zu bilden und andererseits eine genügende Stabilität der Beschichtung in sich.

Zur Beschichtung sind alle biokompatiblen Metalle und Metallegierungen geeignet. Als besonders vorteilhaft haben sich aufgrund ihrer thermischen und mechanischen Eigenschaften Legierungen auf der Basis von Titan, beispielsweise TiAl6V4 und TiAl6Nb7 erwiesen.

Als keramische Werkstoffe für die Lagerschale der Hüftgelenkspfanne können alle in der Prothetik erfolgreich angewendeten Keramikwerkstoffe eingesetzt werden. Als besonders vorteilhaft hat sich aus Gründen der Verschleißfestigkeit, der mechanischen Stabilität und der medizinischen Verträglichkeit das Aluminiumoxid in entsprechender Reinheit bewährt.

Um das Einwachsen des Implantats in den Knochen zu erleichtern und zu beschleunigen, kann die Beschichtung mit einem bioaktiven Überzug versehen sein. Ein für diese Zwecke bekannter Überzug ist beispielsweise Hydroxylapatit. Die Beschichtung erfolgt in der gleichen Weise und in der gleiche Dicke, wie sie beispielsweise bei der Beschichtung der Schäfte angewendet wird, die in den Oberschenkelknochen eingesetzt werden.

An Hand von Ausführungsbeispielen wird die Erfindung näher erläutert.

Es zeigen:
- Figur 1: eine erfindungsgemäße Hüftgelenkspfanne mit einer dichten Beschichtung, deren Oberfläche aufgerauht ist, geschnitten,
- Figur 2: eine erfindungsgemäße Hüftgelenkspfanne, deren Beschichtung porös ist, geschnitten, und
- Figur 3: eine vergrößerte Ansicht des Schnitts durch die Beschichtung der Hüftgelenkspfanne nach Figur 2.

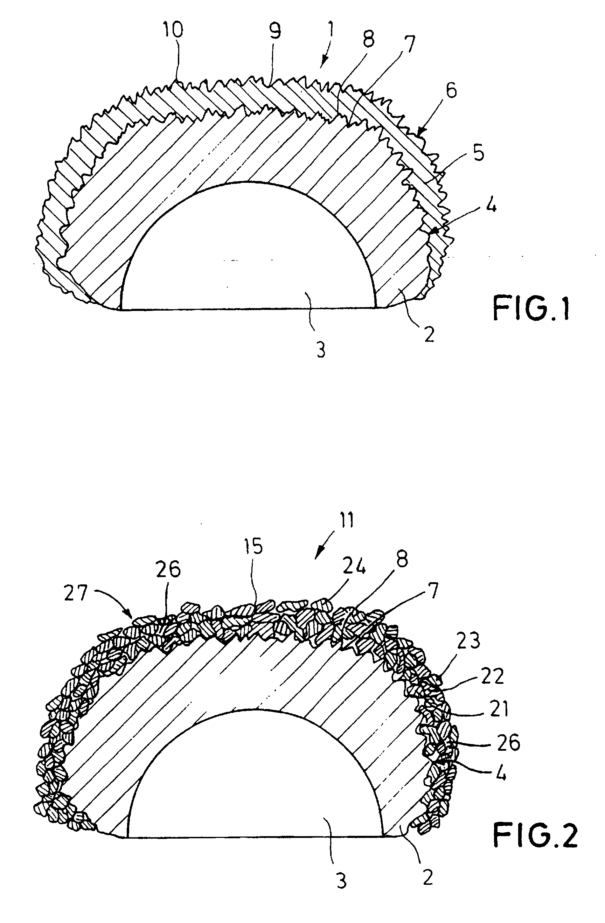

In Figur 1 ist mit 1 insgesamt eine Hüftgelenkspfanne einer Hüftgelenk-Endoprothese bezeichnet. Die Hüftgelenkspfanne 1 ist im Schnitt dargestellt. Die keramische Lagerschale 2 weist eine halbkugelförmige Ausnehmung 3 für die Aufnahme des Kugelkopfs auf. Die Oberfläche 4 des Keramikkörpers 2, die dem Hüftknochen zugewandt ist, ist rauh. In dieser rauhen Oberfläche 4 hat sich die Beschichtung 5 verkrallt. Da das Metall oder die Metallegierung keine metallurgische oder chemische Verbindung mit dem keramischen Werkstoff eingeht, ist eine gute mechanische Verankerung der Beschichtung 5 erforderlich. Die Beschichtung 5 ist deshalb so aufzutragen, daß sie möglichst alle Täler 7 der Oberfläche 4 der Lagerschale 2 ausfüllt und alle Spitzen 8 umhüllt. Im vorliegenden Ausführungsbeispiel ist die Beschichtung aus Metall oder einer Metallegierung dicht. Eine solche dichte Oberfläche kann beispielsweise durch Plasmaspritzen mit entsprechend vorgegebenen Parametern, der Flammentemperatur und des Abstands der Spritzeinrichtung, oder beispielsweise durch Aufdampfen mehrerer Schichten im Vakuum erreicht werden.

Die Beschichtung 5 weist ebenfalls eine rauhe Oberfläche 6 auf. Die rauhe Oberfläche soll eine gute Verankerung des Implantats im Knochen bewirken, indem die Knochensubstanz die Täler 7 ausfüllt und die Spitzen 8 umwächst. Das Einwachsen in die Knochensubstanz kann dadurch beschleunigt werden, daß, wie hier nicht dargestellt ist, die Beschichtung mit einem bioaktiven Überzug versehen ist, wie er beispielsweise bei Schäften der Hüftgelenk-Endoprothesen aufgetragen wird.

Figur 2 zeigt ein weiteres Ausführungsbeispiel einer erfindungsgemäßen Hüftgelenkspfanne 11. Der Schnitt durch die Hüftgelenkspfanne 11 zeigt eine vergleichbare keramische Lagerschale 2 mit der gleichen Ausnehmung 3 für den Kugelkopf und mit einer vergleichbaren rauhen Oberfläche 4. Die Beschichtung 15 unterscheidet sich allerdings im Aufbau von der Beschichtung der Hüftgelenkspfanne des vorhergehenden Ausführungsbeispiels. Die Beschichtung 15 ist porös. Eine poröse Beschichtung kann insbesondere durch Plasmaspritzen eines Metalls oder einer Metallegierung erzeugt werden. Im vorliegenden Ausführungsbeispiel ist auf eine keramische Lagerschale 2 aus Aluminiumoxid eine Titanlegierung TiAl6V4 aufgespritzt. Die Beschichtung 15 besteht im vorliegenden Ausführungsbeispiel aus drei übereinandergespritzten Lagen 21, 22 und 23. Durch Einstellung der Temperatur der Plasmaflamme sowie des Abstands der Spritzeinrichtung von der Oberfläche der Pfanne kann die hier vorliegende Struktur der Beschichtung 15 erzeugt werden. Die Tröpfchen 24 der Metallegierung treffen auf die Oberfläche 4 der keramischen Lagerschale 2 auf. Aufgrund des flüssigen oder teigigen Zustands der Metalltröpfchen werden die Täler 7 der Oberfläche 4 ausgefüllt und die Spitzen 8 umschlossen. Dadurch, daß das Metall oder die Metallegierung mit der Keramik nicht reagiert, erfolgt beim Abkühlen der Tröpfchen 24 eine mechanische Verklammerung der Beschichtung 15 mit der Oberfläche 4 der keramischen Lagerschale 2.

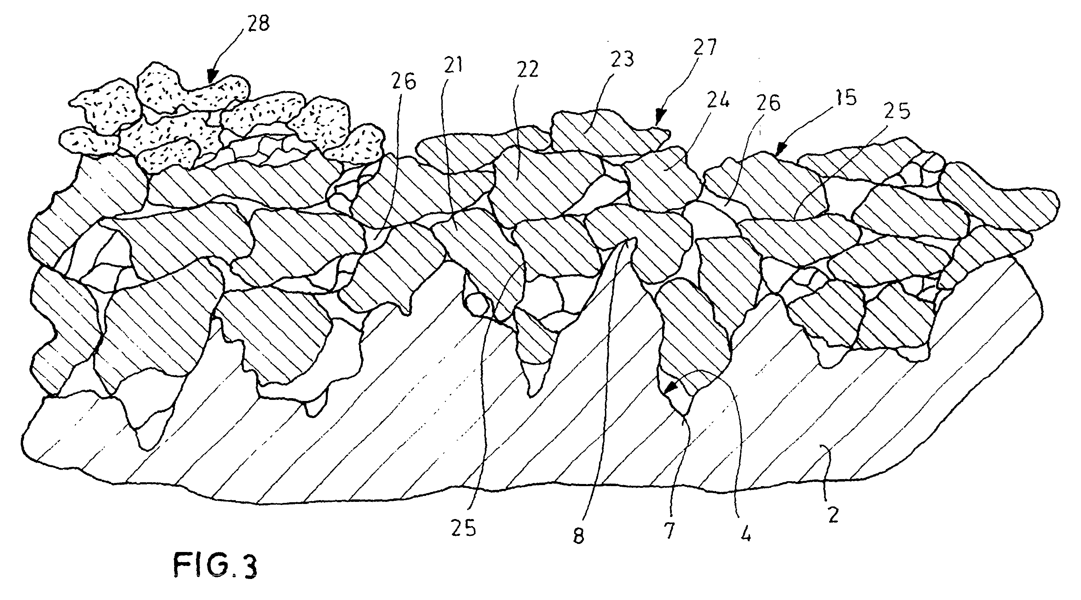

Die Porösität der Beschichtung 15 wird durch eine entsprechende Einstellung des Plasma-Spritzverfahrens erreicht. Die Tröpfchen bilden beim Auftreffen keine dichte Schicht, sondern legen sich übereinander und nebeneinander, wobei sie an den Berührungsflächen 25 (Fig. 3) miteinander verschmelzen oder verschweißen. Wird ein bereits auf der Oberfläche 4 befindliches Tröpfchen durch ein anderes, frisches Tröpfchen getroffen, so kann an der Auftreffstelle aufgrund der mitgeführten Wärmeenergie des frischen Tröpfchens die Oberfläche des ersten Tröpfchens wieder aufgeschmolzen werden und eine entsprechende Verbindung der beiden Tröpfchen an der Berührungsfläche entstehen. Wird, wie im vorliegenden Ausführungsbeispiel, die Beschichtung 15 in mehreren Lagen 21, 22 und 23 aufgespritz, entsteht durch die beschriebene Verbindung der Tröpfchen untereinander eine poröse Schicht, eine Korallenstruktur, wobei zwischen den einzelnen Tröpfchen 24 Poren bzw. Hohlräume 26 verbleiben, in die das Knochengewebe einwachsen kann. Dadurch wird eine gute Verankerung der Beschichtung 15 und damit insgesamt der Hüftgelenkspfanne 1 im Knochengewebe erreicht. Das Einwachsverhalten des Knochens kann, wie hier nicht dargestellt, durch Aufbringen einer bioaktiven Schicht, beispielsweise Hydroxylapatit, weiterhin begünstigt werden.

Figur 3 zeigt einen vergrößerten Ausschnitt aus der Beschichtung 15 der Hüftgelenkspfanne 11. Deutlich zu sehen sind die einzelnen Tröpfchen 24, teilweise oder ganz geschnitten. Die Tröpfchen 24 der ersten Lage 21 haben sich mechanisch mit den Tälern 7 und den Spitzen 8 der rauhen Oberfläche des Keramikwerstoffs der Lagerschale 2 verbunden. Die zweite Lage 22 und die dritte Lage 23 der Beschichtung 15 hat sich nicht nur mechanisch miteinander verklammert sondern an den Berührungsflächen 25 der Tröpfchen 24 sind die Tröpfchen durch Verschmelzen, Verbacken oder Verschweißen auch metallurgisch miteinander verbunden. Die unregelmäßige Struktur der Tröpfchen 24, die insbesondere durch das Aufplatzen der Tröpfchen beim Auftreffen entsteht, führt ebenfalls zu Poren und Hohlräumen 26. Zusätzlich zu diesen Poren und Hohlräumen bietet sich dem Knochengewebe auch an der insgesamt rauhen Oberfläche 27 der Beschichtung 15 eine gute Einwachs- und Verankerungsmöglichkeit.

In Figur 3 ist ein Teil der Beschichtung 15 zusätzlich mit einem bioaktiven Überzug 28 beschichtet. Diese bioaktiven Überzüge, beispielsweise aus Hydroxylapatit, sind bekannt und werden in der Regel durch Spritzen aufgetragen, so daß sich auch hier eine Oberflächenstruktur bildet, die das Einwachsen des Knochengewebes begünstigt.

## Patentansprüche

1. Hüftgelenkspfanne einer Hüftgelenk-Endoprothese, bei der die den Kugelkopf des Schaftes lagernde Lagerschale der Pfanne aus einem keramischen Werkstoff besteht, **dadurch gekennzeichnet, daß** die Lagerschale (2) der Hüftgelenkspfanne (1; 11) in dem Bereich ihrer Oberfläche (4), mit dem sie in den Hüftknochen eingesetzt wird, mit einer Beschichtung (5; 15) aus einem biokompatiblen Metall oder einer biokompatiblen Metallegierung überzogen ist.

2. Hüftgelenkspfanne nach Anspruch 1, **dadurch gekennzeichnet, daß** die zu beschichtende Oberfläche (4) der Lagerschale (2) der Hüftgelenkspfanne (1; 11) zur Haftverbesserung der Beschichtung (5; 15) aufgerauht ist.

3. Hüftgelenkspfanne nach Anspruch 1 oder 2, **dadurch gekennzeichnet, daß** die Beschichtung (5) aufgedampft ist.

4. Hüftgelenkspfanne nach Anspruch 1 oder 2, **dadurch gekennzeichnet, daß** die Beschichtung (15) aufgespritzt ist.

5. Hüftgelenkspfanne nach einem der Ansprüche 1 bis 4, **dadurch gekennzeichnet, daß** die Beschichtung (15) aus mehreren Lagen (21, 22, 23) besteht.

6. Hüftgelenkspfanne nach einem der Ansprüche 1 bis 5, **dadurch gekennzeichnet, daß** die Beschichtung (5; 15) auf ihrer Oberfläche (6; 27) rauh ist.

7. Hüftgelenkspfanne nach einem der Ansprüche 4 bis 6, **dadurch gekennzeichnet, daß** die Beschichtung (15) porös ist.

8. Hüftgelenkspfanne nach einem der Ansprüche 1 bis 7, **dadurch gekennzeichnet, daß** die Dicke der Beschichtung (5; 15) dünner als 1 mm ist.

9. Hüftgelenkspfanne nach Anspruch 8, **dadurch gekennzeichnet, daß** die Dicke der Beschichtung (5; 15) zwischen 50 Mikrometern und 150 Mikrometern liegt.

10. Hüftgelenkspfanne nach einem der Ansprüche 1 bis 9, **dadurch gekennzeichnet, daß** die Beschichtung (5; 15) aus einer Titanlegierung besteht.

11. Hüftgelenkspfanne nach einem der Ansprüche 1 bis 10, **dadurch gekennzeichnet, daß** die Beschichtung (5; 15) mit einem bioaktiven Überzug (28) versehen ist.

12. Hüftgelenkspfanne nach einem der Ansprüche 1 bis 11, **dadurch gekennzeichnet, daß** die Lagerschale (2) der Hüftgelenkspfanne (1) aus Aluminiumoxid besteht.

## Claims

1. Hip-joint socket of a hip-joint endoprosthesis, in which the bearing shell of the socket that mounts the ball head of the shaft is made of a ceramic material, **characterised in that** the bearing shell (2) of the hip-joint socket (1; 11), in the region of its surface (4) with which it is inserted into the hip bone, is covered with a coating (5; 15) that is made of a biocompatible metal or a biocompatible metal alloy.

2. Hip-joint socket according to claim 1, **characterised in that** the surface (4) of the bearing shell (2) of the hip-joint socket (1; 11) to be coated is roughened in order to improve the adhesion of the coating (5; 15).

3. Hip-joint socket according to claim 1 or 2, **characterised in that** the coating (5) is vapour-deposited thereon.

4. Hip-joint socket according to claim 1 or 2, **characterised in that** the coating (15) is sprayed thereon.

5. Hip-joint socket according to one of claims 1 to 4, **characterised in that** the coating (15) consists of a plurality of layers (21, 22, 23).

6. Hip-joint socket according to one of claims 1 to 5, **characterised in that** the coating (5; 15) is rough on its surface (6; 27).

7. Hip-joint socket according to one of claims 4 to 6, **characterised in that** the coating (15) is porous.

8. Hip-joint socket according to one of claims 1 to 7, **characterised in that** the thickness of the coating (5; 15) is thinner than 1 mm.

9. Hip-joint socket according to claim 8, **characterised in that** the thickness of the coating (5; 15) lies between 50 micrometres and 150 micrometres.

10. Hip-joint socket according to one of claims 1 to 9, **characterised in that** the coating (5; 15) is made of a titanium alloy.

11. Hip-joint socket according to one of claims 1 to 10, **characterised in that** the coating (5; 15) is provided with a bioactive covering (28).

12. Hip-joint socket according to one of claims 1 to 11, **characterised in that** the bearing shell (2) of the hip-joint socket (1) is made of aluminium oxide.

## Revendications

1. Cotyle d'endoprothèse de hanche dans lequel la cupule du cotyle recevant la tête sphérique de la tige est réalisée en un matériau céramique, **caractérisé en ce que** la cupule (2) du cotyle prothétique (1; 11), dans la partie de sa surface (4) par laquelle elle est insérée dans l'os de la hanche est recouverte d'un revêtement (5; 15) en un matériau biologiquement compatible ou en un alliage de métal biologiquement compatible.

2. Cotyle prothétique de hanche selon la revendication 1, **caractérisé en ce que** la surface (4) à revêtir de la cupule (2) du cotyle prothétique de hanche (1 ; 11) est rendue rugueuse aux fins d'améliorer l'adhérence du revêtement (5 ; 15).

3. Cotyle prothétique de hanche selon la revendication 1 ou 2, **caractérisé en ce que** le revêtement (5) est appliqué par métallisation.

4. Cotyle prothétique de hanche selon la revendication 1 ou 2, **caractérisé en ce que** le revêtement (5) est appliqué par pulvérisation.

5. Cotyle prothétique de hanche selon une des revendications 1 à 4, **caractérisé en ce que** le revêtement (15) est formé de plusieurs couches (21, 22, 23).

6. Cotyle prothétique de hanche selon une des revendications 1 à 5, **caractérisé en ce que** le revêtement (5 ; 15), au niveau de sa surface (6 ; 27), est rugueux

7. Cotyle prothétique de hanche selon une des revendications 4 à 6, **caractérisé en ce que** le revêtement (15) est poreux.

8. Cotyle prothétique de hanche selon une des revendications 1 à 7, **caractérisé en ce que** l'épaisseur du revêtement (5 ; 15) est inférieure à 1 mm.

9. Cotyle prothétique de hanche selon la revendication 8, **caractérisé en ce que** l'épaisseur du revêtement (5 ; 15) est comprise entre 50 micromètres et 150 micromètres.

10. Cotyle prothétique de hanche selon une des revendications 1 à 9, **caractérisé en ce que** le revêtement (5 ; 15) est en un alliage de titane.

11. Cotyle prothétique de hanche selon une des revendications 1 à 10, **caractérisé en ce que** le revêtement (5 ; 15) est pourvu d'une couche bioactive (28).

12. Cotyle prothétique de hanche selon une des revendications 1 à 11, **caractérisé en ce que** la cupule (2) du cotyle prothétique (1) de hanche est en oxyde d'aluminium.
